# FASCICULE DE BREVET EUROPEEN

(11) **EP 1 389 118 B1**
(45) Date de publication et mention de la délivrance du brevet: **15.07.2009**
(21) Numéro de dépôt: 02743328.3
(22) Date de dépôt: 23.05.2002
(51) Int. Cl.: A61K 31/155, A61K 31/64, A61K 9/50

(54) **FORME PHARMACEUTIQUE ORALE ANTIDIABETIQUE "UNE PRISE PAR JOUR" COMPRENANT UNE BIGUANIDE ET AU MOINS UN AUTRE PRINCIPE ACTIF**
ORAL ANZUWENDENDE ZUCKERKRANKHEITSHEMMENDE "EINMAL TÄGLICH" ARZNEIZUSAMMENSETZUNG ENTHALTEND EINE BIGUANIDVERBINDUNG UND ZUMINDESTENS EINEN ANDEREN WIRKSTOFF
SINGLE-DAILY-DOSE ANTIDIABETIC ORAL PHARMACEUTICAL FORM COMPRISING A BIGUANIDE AND AT LEAST ANOTHER ACTIVE PRINCIPLE

(30) Priorité: 23.05.2001 FR 0106854
(43) Date de publication de la demande: 18.02.2004
(73) Titulaire: FLAMEL TECHNOLOGIES, 69200 Vénissieux (FR)
(72) Inventeur: CASTAN, Catherine, F-69530 ORLIENAS (FR); SOULA, Gérard, F-69330 Meyzieu (FR); MEYRUEIX, Rémi, F-69009 Lyon (FR)
(74) Mandataire: Fleurance, Raphaël
(86) Numéro de dépôt international: PCT/FR2002/001745
(87) Numéro de publication internationale: WO 2002/094285

(56) Documents cités:
- EP-A1- 0 974 356
- WO-A1-00/40233
- WO-A1-97/17975
- WO-A2-01/21159
- WO-A2-01/32157
- WO-A2-01/32158
- WO-A2-96/11675
- US-A- 6 099 862

## Description

L'invention concerne des formes galéniques orales (comprimés, gélules, poudres...) dans lesquelles sont associés plusieurs principes actifs antidiabétiques et dont le régime journalier d'administration est le plus réduit possible, de préférence égal à une prise par jour.

Les principes actifs concernés sont des antidiabétiques, et plus précisément un biguanide, à savoir la metformine.

L'intention vise une forme galénique orale d'antidiabétique (diabète type II) comprenant un principe actif A, la metformine, A étant associé à au moins un autre principe actif B antihyperglycémiant.

Les pathologies auxquelles on s'intéresse plus particulièrement dans le cadre de l'invention, sont les diabètes de type II non insulinodépendants. Dans ces diabètes de type II, on observe chez les patients des hyperglycémies qui trouvent leur origine dans une déficience de sécrétion d'insuline par les cellules β du pancréas, en conjugaison avec une résistance à l'insuline et une tolérance au glucose réduite.

Le diabète, et en particulier le diabète de type II, est une maladie chronique qui entraîne des complications graves, notamment sur le plan microvosasculaire, neurologique et macrovasculaire.

Parmi les complications microvasculaires, on peut citer les dysfonctionnements des vaisseaux capillaires sanguins au niveau de la rétine, des reins et des nerfs. Les rétinopathies et les néphropathies sont des séquelles bien connues du diabète non insulinodépendant.

Les complications neurologiques ont pour cadre le système nerveux périphérique (paralysie, douleur, déficit sensoriel, atrophie musculaire...) et le système nerveux autonome (diarrhées diabétiques, pertes des réflexes cardiovasculaires, troubles de la vessie et de l'estomac, impuissnnce...).

Les complications macrovasculaires sont notamment l'athérosclérose coronaire cérébrale et périphérique et les maladies cardiaques coronariennes.

Le diabète, et en particulier le diabète de type II est une pathologie chronique très sévère qui peut être létale.

Le traitement de cette maladie repose sur trois bases :
a) administration quotidienne de médicaments antidiabétiques,
b) régime alimentaire contrôlé,
c) exercice physique.

Le traitement thérapeutique le plus commode et le moins pénible pour les patients, est sans conteste un traitement par voie orale.

Les antidiabétiques oraux (antihyperglycémiants) sont choisis dans les familles suivantes :
- LES SULFONYLURÉS :
   le glibenclamide, le nateglinide, le glimepiride, le glipizide, le gliclazide, le tolbutamide, le tolazamide, la gliquidone et le chlorpropamide étant plus spécifiquement retenus ;
- LES THIAZOLIDINEDIONES :
   le maléate de rosiglitazone, la troglitazone (US-A-4 572 912), le zorglitazone, l'englitazone, la darglitazone, le produit MITSUBISHI MCC-555 (US-A-5 594 016), le produit GLAXO-WELCOME GL-262570 et le chlorhydrate de pioglitazone étant plus spécifiquement retenus ;
- LES BIGUANIDES ;
   la metformine étant plus spécialement retenue ;
- LES INHIBITEURS D'α-GLUCOSIDASE ;
   l'acarbose (US-A-4 904 769) ou le miglitol (US-A-4 639 436) ;
- LES METIGLINIDES ;
   le repaglinide :
- LES INSULINES :
- ET LEURS ASSOCIATIONS.

Les sulfonylurées et les biguanides sont les antidiabétiques oraux majeurs.

Les sulfonylurées agissent en simulant la sécrétion de l'insuline. Leurs cibles sont les cellules β du pancréas productrices d'insuline.

Les biguanides tels que la metformine inhibent la glycogénèse et augmentent l'utilisation périphérique du glucose. Les biguanides ne peuvent être actifs qu'en présence d'insuline endogène.

Depuis l'introduction de ces différents médicaments antidiabétiques, les médecins prescrivent notamment des traitements oraux du diabète qui combinent ces différents produits. Cela contraint les patients à prendre ces combinaisons de médicaments plusieurs fois par jour. Inévitablement, on observe alors une faible observance (respect de la posologie) de la part des patients, qui sont souvent des personnes âgées. Dans ces conditions, les traitements oraux n'ont pas les effets escomptés et les patients subissent les complications graves, rappelées ci-dessus et propres aux diabètes (notamment de type II).
Ainsi, pour ces maladies chroniques graves telles que le diabète de type II, il est clair que l'observance est un paramètre fondamental pour l'efficacité du traitement (prévention des graves désordres engendrés par l'hyperglycémie et survie du patient).
En améliorant l'observance, on limiterait les erreurs de posologie et leur cortège d'effets délétères. De surcroît, le confort des patients n'en serait que meilleur.

On mesure ainsi l'importance essentielle qu'aurait pour les malades, une solution thérapeutique et galénique simplifiée, limitée à une prise orale par jour, d'une combinaison de plusieurs médicaments antidiabétiques complémentaires, validés par l'usage et les études cliniques.

Cependant pour parvenir à cette solution idéale, il convient de résoudre bon nombre de problèmes techniques, dont certains sont indiqués ci-après.

Le premier problème auquel se trouvent confrontés les chercheurs est celui des différences entre les régimes quotidiens d'administration des antidiabétiques oraux. Cela constitue un frein aulx associations de différents principes actifs dans une même forme galénique orale.

En effet, s'agissant d'un biguanide comme la metformine, on a affaire à un principe actif antidiabétique, dont la forme à libération immédiate a un régime d'administration de deux prises quotidiennes, alors que d'autres classes d'antidiabétiques tels que les sulfonylurées (glibenclamide) sont administrées oralement à raison d'une fois par jour.

On comprend alors que, dans cet exemple, on ne peut associer la metformine et le glibenclamide dans une même forme galénique orale, qu'à la condition d'augmenter la durée d'action in vivo (biodisponibilité) de la metformine, de façon à ramener le régime d'administration de la metformine à une prise par jour, sans modifier le comportement du principe actif associé, en l'occurrence le glibenclamide.

Un autre préalable à la solution thérapeutique et galénique susvisée, passe par la mise en oeuvre de l'association de deux principes actifs compatibles, de manière à garantir la stabilité au stockage de la forme pharmaceutique considérée. Les deux principes actifs ne doivent pas être sujets à des interactions conduisant à des dégradations.

Un autre point galénique critique est de minimiser les phénomènes de libération de doses massives de principes actifs, de manière locale et prolongée, dans le tractus gastro-intestinal ("dose dumping"). Ces phénomènes sont responsables de troubles gastro-intestinaux graves, tels que des ulcérations gastriques.

Le goût désagréable de certains principes actifs est de nature à perturber l'observance chez certains patients. Il est donc important d'offrir des solutions galéniques permettant un masquage du goût des principes actifs.

Il convient également que la forme pharmaceutique orale (comprimé, gélule, poudre ou sachet) que l'on cherche à élaborer, soit facile à avaler, y compris pour les personnes âgées.

Il existe, dans ce contexte, un certain nombre de propositions techniques antérieures, qui ont cherché, en vain, à résoudre la problématique susdécrite.

Ainsi, la demande de brevet européen EP-A-0 974 356 décrit des comprimés comprenant une combinaison de metformine et de 20 glibenclamide, dans lesquels la taille des particules de glibenclamide est telle qu'au plus 10% des particules ont une taille inférieure à 2 µm et qu'au plus 10% de ces particules ont une taille supérieure à 60 µm.
Le comprimé de metformine/glibenclamide est obtenu par compression de :
- granulés à base de polyvinylpyrrolidone (66,6 g), de metformine (1500 g), de glibenclamide (16,5 g avec 10 à 90 % des particules de taille comprise entre 2 et 60 µm), de croscarmellose de sodium (42 g) et de cellulose microcristalline (284,4 g) ;
- cellulose microcristalline (97,5 g) ;
- stéarate de magnésium (12 g).
Les comprimés sont ensuite enrobés avec de l'hydroxypropyl-méthylcellulose. L'inconvénient majeur de ces comprimés tient à leur régime quotidien d'administration, qui est de deux prises par jour, à cause de la metformine, et ce malgré le fait que le glibenclamide seul peut être ingéré à raison d'une fois par jour.
Ce comprimé est donc perfectible au regard de l'amélioration de l'observance.
De plus, il est à craindre que les principes actifs nus, non protégés (metformine/glibenclamide) contenus dans ce comprimé selon l'EP-A-0 974 356, interagissent et se dégradent prématurément au stockage, avant d'être ingérés.

On connaît également par le brevet US-A-6 099 862 un comprimé phamaceutique à libération contrôlée contenant un principe actif antihyperclycémiant et un principe actif hypoglycémiant. Ce comprimé comprend un coeur contenant les principes actifs, un revêtement membranaire sem-perméalable entourant le coeur et au moins une voie de passage dans cette membrane pour permettre aux principes actifs d'être libérés. Plus particulièrement, ce document décrit des comprimés comprenant un coeur constitué de metformine et de glipizide. Là encore, il est à craindre que les principes actifs contenus dans ces comprimés, interagissent et se dégradent prématurément au stockage, avant d'être ingérés.

On connaît également par la demande WO 96/11675 des microcapsules de type réservoir contenant au moins un principe actif, constituées par des particules de principes actifs recouvertes chacune d'une pellicule d'enrobage présentant une composition spécifique constituée d'au moins un polymère filmogène, d'au moins un polymère azoté, d'au moins un plastifiant et d'au moins un agent tensioactif et/ou lubrifiant, Selon ce document, les principes actifs sont combinés dans une même microcapsule. De plus, ce document ne donne aucun enseignement concret sur l'utilisation de deux principes actifs différents, présentant des régimes quotidiens d'administration différents, dans une même formulation de sorte que la forme galénique obtenue soit prise une seule fois par jour.

On connaît également par la demande WO-99/47128, un système biphasique pour la libération contrôlée de metformine. Cette forme galénique ne comprend qu'un seul principe actif : la metformine. Le système galénique considéré est constitué par un comprimé comprenant une phase externe matricielle faite d'hydroxypropylméthylcellulose et de cellulose microcristalline. Sont inclus dans cette phase externe, des granulés qui forment la phase interne et qui sont constitués par de la metformine et de l'éthycellulose ou de la carboxyméthylcellulose.
Ce système galénique est conçu pour avoir un temps de séjour prolongé dans l'estomac, sans se désagréger.
Il existe ainsi un risque non négligeable de libération massive de la metformine sur une zone limitée de la paroi stomacale ("dose dumping"). Or ce phénomène est générateur de troubles gastriques tout à fait indésirables pour le patient. Cet inconvénient est d'autant moins tolérable, dans un traitement permanent pour une maladie chronique.
Ce système biphasique matriciel serait impropre à accueillir un autre principe actif antidiabétique, associé à la metformine. En effet, cela serait une gageure de réussir à maîtriser la cinétique de libération du principe actif supplémentaire, pour l'harmoniser avec la cinétique de libération de la metformine. Dans ces conditions, il semble a priori très délicat, d'obtenir un régime d'administration d'une prise quotidienne, pour les deux principes actifs.
En résumé, l'enseignement de ce document ne s'inscrit pas dans le contexte d'association d'un biguanide (metformine) A et d'au moins un outre antihyperglycémiant B, dans une même forme galénique orale, à une seule prise par jour. En outre, il ne résout pas la plupart des éléments de la problématique décrite supra.

Il en va de même pour les demandes PCT WO-A-98/55107 & WO-A-99/47125, qui concernent aussi des systèmes galéniques monolithiques, de la taille d'un comprimé (≈ 10 mm), ne contenant que la metformine à titre de principe actif antidiabétique.

Dans cet état de la technique, l'un des objectifs essentiels de la présente invention est de résoudre la problématique mentionnée ci-avant, qui est de fournir une forme pharmaceutique orale antidiabétique (diabète type II) :
- contenant un principe actif A formé par de la metformine et au moins un autre principe actif B,
- et susceptible d'être avalée aisément, à raison d'une fois par jour.

Un autre objectif essentiel de l'invention est de fournir une forme pharmaceutique orale à base de metformine associée à un autre principe actif B antidiabétique (promoteur de l'action de A), permettant une simplification et une amélioration de l'observance, sans que ces gains ne se fassent au détriment de l'efficacité thérapeutique.

Un autre objectif essentiel de l'invention est de fournir une forme pharmaceutique orale, "une prise par jour", à base de metformine associée à au moins un autre principe actif B antidiabétique promoteur de l'action de A, sans négliger les effets gastriques indésirables ("dose dumping") et économiques, dans cette recherche d'une solution galénique à la problématique susmentionnée.

Un autre objectif essentiel de l'invention est de fournir une forme pharmaceutique orale, "une prise par jour", à base de metformine associée à un autre principe actif B antihyperglycemiant en ayant recours à des auxiliaires pharmaceutiques (excipients) inoffensifs et approuvés comme tels, par les autorités réglementaires.

Un autre objectif essentiel de l' invention est de fournir une forme pharmaceutique orale, "une prise par jour", à base de metformine associée à un autre principe actif B antihyperglycémiant, qui soit facile à avaler.

Un autre objectif essentiel de l'invention est de fournir un moyen de polythérapie (bithérapie) du diabète (en particulier de type II) comprenant la metformine et au moins un autre principe actif B et se présentant sous forme d'une entité galénique administrée une fois par jour, dans laquelle sont évitées les interactions délétères entre la metformine et le principe actif B, lors du stockage.

Un autre objectif essentiel de l'invention est de fournir une forme galénique orale à base de metformine et d'au moins un autre principe actif B, dans laquelle le goût de A et éventuellement de B est masqué.

Ces objectifs, parmi d'autres, sont atteints par l'invention qui concerne une forme pharmaceutique orale telle que décrite dans la revendication 1 comprenant notamment une association :
➢ d'un principe actif A constitué par un biguanide, à savoir la metformine,
➢ et d'au moins un autre principe actif B différent de A et choisi parmi les antidiabétiques, de préférence les antihyperglycémiants, dont le régime d'administration est d'une ou plusieurs prises par jour,
**caractérisée:**
**❖ en ce qu**'elle comporte ;
   - une pluralité de capsules constituées chacune par un coeur à base de metformine et par une pellicule d'enrobage appliquée sur le coeur et permettant la libération prolongée in vivo de la metformine ;
   - et éventuellement dans le cas où le régime d'administration du principe actif B est égal à plusieurs prises par jour, une pluralité de capsules constituées chacune par un coeur à base de principe actif B et par une pellicule d'enrobage appliquée sur le coeur et permettant la libération prolongée in vivo du principe actif B ;
❖ et en ce que les capsules à base de A et les éventuelles capsules à base de B, sont conçues de telle sorte que le régime d'administration de la forme galénique considérée soit d'une prise par jour.

Conformément à l'invention, on est avantageusement parvenu à mettre au point une forme galénique orale antidiabétique à base de metformine et d'au moins un autre principe actif antidiabétique B, dans laquelle le régime quotidien d'administration de la metformine, et éventuellement le régime quotidien d'administration du principe actif B, a (ont) été ajustée(s) à une seule prise par jour, grâce à la mise en oeuvre de capsules à base de A, voire de capsules à base de B, enrobées et individualisées. Cet enrobage a une structure et une composition qui permettent le réglage de la libération prolongée in vivo du principe actif A. voire B, et donc indirectement l'allongement de la durée d'action de A, voire de B.

Ainsi, si le régime d'administration de A est de deux prises par jour, tandis que celui de B est d'une prise par jour, on met la metformine (A) sous forme de capsules enrobées et on l'associe au principe actif B. Ce dernier est exempt de transformation de nature à modifier sa vitesse de libération in vivo et sa biodisponibilité. Il est alors possible de préparer des comprimés, des gélules ou des sachets de poudre comprenant les doses quotidiennes requises en A et B, et dont l'ingestion une seule fois par jour est aisée, ce qui optimise l'observance.

Au surplus, le fait que la metformine (A), voire le principe actif B, soit encapsulée, permet d'éviter toute éventuelle interaction nuisible entre A et B, lors du stockage.

Par ailleurs, les formes galéniques selon l'invention ne sont pas des formes galéniques monolithiques de grandes tailles susceptibles de se trouver bloquées dans les méandres du tractus gastro-intestinal, en risquant ainsi d'être à l'origine d'une libération massive et très localisée des principes actifs A et B ("dose dumping"). Dans ce cas de figure, les principes actifs A et B ne sont non seulement pas absorbés selon les profils recherchés, mais de surcroît sont susceptibles de provoquer des lésions locales graves.

Un autre avantage de la forme galénique antidiabétique A, B selon l'invention est de permettre grâce à l'enrobage régissant la libération prolongée, le masquage du goût de la metformine, voire du ou des principes actifs B.

Enfin et surtout, des régimes journaliers d'administration d'une prise par jour, sont parfaitement conformes aux attentes en matière d'observance et donc de respect des posologies, ce qui est important dans cette maladie chronique qu'est le diabète, et en particulier le diabète de type II.

Les capsules à base de A et les éventuelles capsules à base de B, sont des microcapsules. Ces microcapsules sont caractérisées par une granulométrie comprise entre 50 et 1000 µm, de préférence entre 100 et 750 µm, et, plus préférentiellement encore, entre 200 et 500 µm.

L'enrobage des capsules est élément important de la forme pharmaceutique selon la présente invention, puisqu'il régit la cinétique de libération prolongée, in vivo, des principes actifs A, voire B, contenus dans le coeur des capsules. In fine, l'enrobage détermine la durée d'action des principes actifs A, voire B, et donc le régime d'administration quotidien d'une prise/jour.
La composition de cet enrobage est donc cruciale.

Cette composition de la pellicule d'enrobage des capsules à base de metformine et des éventuelles capsules à base de B, est la suivante :
1) - au moins un polymère filmogène (P1) insoluble dans les liquides du tractus, présent à raison de 50 à 90, de préférence 50 à 80 % en poids sur sec par rapport à la masse totale de la composition d'enrobage et constitué par au moins un dérivé non hydrosoluble de la cellulose, à savoir l'éthylceltulose et/ou l'acétate de cellulose ;
2) - au moins un polymère azoté (P2) présent à raison de 2 à 25, de préférence 5 à 15 % en poids sur sec par rapport à la masse totale de la composition d'enrobage et constitué par au moins un polyacrylamide et/ou un poly-N-vinylamide et/ou un poly-N-vinyi-lacteme, à savoir le polyacrylamide et/ou la polyvinylpyrrolidone ;
3) - au moins un plastifiant présent à raison de 2 à 20, de préférence de 4 à 15 % en poids sur sec par rapport à la masse totale de la composition d'enrobage et constitué par au moins l'un des composés suivants : les esters du glycérol, les phtalates, les citrates, les sébaçates, les esters de l'alcool cétylique, l'huile de ricin ;
4) - et éventuellement au moins un agent tensioactif et/ou lubrifiant, présent à raison de 2 à 20, de préférence de 4 à 15 % en poids sur sec par rapport à la masse totale de la composition d'enrobage et choisi parmi les tensioactifs anioniques, à savoir les sels alcalins ou alcalinoterreux des acides gras, l'acide stéarique et/ou oléique étant préférés, et/ou parmi les tensioactifs non ioniques, à savoir les esters de sorbitan polyoxyéthylénés et/ou les dérivés de l'huile de ricin polyoxyéthylénés, et/ou parmi les agents lubrifiants comme les stéarates de calcium, de magnésium, d'aluminium ou de zinc, ou comme le stéarylfumarate de sodium et/ou le béhénate de glycérol; ledit agent pouvant comprendre un seul ou un mélange des susdits produits.

Un tel enrobage permet de régler de manière indépendante, la cinétique de libération in vivo de A et éventuellement de B. Cela est possible dans la nouvelle forme galénique selon l' invention, car les capsules discrètes et individualisées de A enrobé, sont simplement juxtaposées physiquement avec B sous forme ou non de capsules. Ces capsules de A enrobé et le (ou les) principe(s) actif(s) B encapsulé(s), ont en effet des cinétiques de libération et d'absorption in vivo, qui leur sont propres et qui sont différentes l'une de l'autre.

Ce système *multi(micro)capsulaire* a pour autre avantage d'offrir un masquage du goût de A, voire de B au besoin, ainsi que toute la sécurité souhaitable au regard du phénomène de "dose dumping".

Le médicament selon l'invention est particulièrement adapté aux principes actifs anti-hyperglycémiants qui ont pour caractéristique d'avoir une fenêtre d'absorption située dans les parties hautes du tractus gastro-intestinal (estomac et début intestin grêle), qui sont très solubles dans l'eau et dont la posologie est de l'ordre de 1g à 2g par jour, ce qui impose l'ingestion d'une grande masse de produit par prise.

Ce médicament sous une forme galénique "*multi(micro)capsulaire*" composée d'une pluralité de capsules, favorise, pour des raisons statistiques, une bonne absorption dans la fenêtre d'absorption et supprime le risque d'accumulation localisée de principe actif. Il en résulte une absorption optimale d'anti-hyperglycémiants dans la fenêtre d'absorption, en quantité et sur une durée telles que la couverture thérapeutique peut être garantie sur au moins 12h, avec toute l'efficacité thérapeutique souhaitable (contrôle de la glycémie). En effet, le grand nombre de particules (e.g. de l'ordre de 10 000 pour les microcapsules et de 100 pour les macroparticules) permet une distribution reproductible, diminuant ainsi les risques d'hyper et d' hypoglycémie.

De manière plus préférée encore, l'enrobage des capsules de A des éventuelles capsules B, a la composition suivante :
-1- Ethylcellulose
-2- Polyvinylpyrrolidone
-3- Huile de ricin
-4- Stéarate de magnésium.

Il est à noter que dans le cas où le ou les principe(s) actif(s) B ont un régime d'administration d'une prise par jour, il n'est (ne sont) pas revêtu(s) d'un enrobage permettant une libération prolongée et contrôlée. Il s'agit d'un principe actif à libération immédiate. Pour des raisons tenant non pas à la cinétique de libération, mais à la formulation galénique, ce principe actif peut être néanmoins enveloppé dans un enrobage de protection, sans effet sur la cinétique de libération immédiate. Un tel enrobage neutre est par exemple constitué de :
❖ sucres tels que sucrose, glucose, lactose, maltitol,
mannitol, isomalt, sorbitol, xylitol, hydrolysats d'amidon,
❖ gélatine,
❖ alginate,
❖ gommes telles que la gomme d'acacia,
❖ les cires telle la cire de carnauba,
❖ l'alcool polyvinylique,
❖ les polyethylèneglycols,
❖ les poloxamers,
❖ la polyvinylpyrrolidone,
❖ l'hydroxypropylméthylcellulose, la méthylcellulose,
l'hydroxypropylcellulose, la carboxyméthylcellulose, la méthylcellulose, l'acétate/phtalnte de cellulose, le phtalate d'hydroxypropylméthylcellulose, l'acétate-succinate d'hydroxypropylméthylcellulose,
❖ l'Eudragit E, etc...
Cela renvoie à des pratiques galéniques usuelles.

La forme galénique selon l' invention peut également être définie par des caractéristiques de libération in vitro du ou des principe(s) actif(s) anti-hyperglycémiant(s). D'où il s'ensuit que dans un test de dissolution in vitro dénommé *dissolutest* de type II conformément à la pharmacopée, la dissolution du ou des principe(s) actif(s) anti-hyperglycémiant(s) s'étend sur au moins 8 heures, de préférence au moins 20 heures.

Pour détailler quelque peu la structure des capsules, on précise que le coeur desdites capsules peut être par exemple :
✔ un granulé comportant du principe actif anti-hyperglycémiant et des excipients de granulation
✔ et/ou une particule de principe actif anti-hyperglycémiant, de préférence un monocristal.

Dans le coeur des capsules, le principe actif anti-hyperglycémiant peut être associé à un ou plusieurs excipients. C'est notamment le cas lorsque le coeur est constitué par un granulé. Les excipients et les techniques de granulation mis en oeuvre, sont ceux qui sont traditionnels dans la granulation.

Les excipients de granulation mis en oeuvre sont bien connus de l'homme de l'art et sont notamment ceux exemplifiés ci-dessus.
En pratique, le pelliculage déposé sur chaque granulé peut être constitué par une ou plusieurs macromolécules filmogènes du type de celles mentionnées ci-dessus.

L'homme de l'art connaît plusieurs technologies de pelliculage. A titre d'exemple, on peut citer celle faisant intervenir un système WURSTER® de la société GLATT ou un système PRECISIONCOATER® de la société AEROMATIC. -

S'agissant du principe actif A, on désigne par le terme "metformine", la metformine et ses sels, tels que le chlorhydrate de metformine.

Concernant le (ou les) principe(s) actif(s) B, il(s) est (sont) choisi(s), à titre non limitatif, dans le groupe de familles d'agents antihyperglycémiants comprenant :
- LES SULFONYLURÉES ;
   le glibenclamide, le nateglinide, le glimepiride, le glipizide, le gliclazide, le tolbutamide, le tolozamide, la gliquidone et le chlorpropamide étant plus spécifiquement retenus ;
- LES THIAZOLIDINEDIONES ;
   le maléate de rosiglitazone, la troglitazone, le zorglitazone, l'englitazone, la darglitazone, le produit MITSUBISHI MCC-555, le produit GLAXO-WELCOME GL-262570 et le chlorhydrate de pioglitazone étant plus spécifiquement retenus ;
- LES INHIBITEURS D'α-GLUCOSIDASE ;
   l'acarbose ou le miglitol ;
- LES METIGLINIDES ;
   le repaglinide ;
- LES INSULINES ;
- ET LEURS ASSOCIATIONS.

Le système galénique selon l'invention peut se présenter sous forme d'une unité galénique de masse et de volume adaptés pour permettre, à chaque administration orale journalière, l'absorption des doses respectives d_{A} et d_{B} quotidiennes requises en principes actifs A et B.

En pratique, la formé pharmaceutique orale multi-microcapsulaire selon l'invention est constituée par un comprimé, une gélule ou de la poudre (conditionnée en sachet). Ces unités galéniques comprennent comme constituants essentiels, dissociés physiquement et sur le plan de la cinétique de libération, des capsules de metformine (A) et des particules de B sous forme ou non de capsules.

Plus précisément, les unités galéniques concernées peuvent être, notamment :
- des comprimés délitables dans la bouche,
- des comprimés effervescents,
- des comprimés délitables dans un liquide (eau),
- des poudres conditionnées en sachet de doses données,
- des suspensions de capsules dans un liquide (eau),
- ou des gélules contenant une poudre de capsules.

Pour illustrer l'invention sur le plan quantitatif, on peut citer à titre d'exemples; des unités galéniques à base de capsules de metformine, dans lesquelles :
- quand B = glibenclamide :
   les doses quotidiennes requises pour A et B,
   respectivement d_{A} et d_{B} sont telles que :
   250 mg ≤ d_{A} ≤ 2000 mg
   1,25 mg ≤ d_{B} ≤ 20 mg
   par exemple
   d_{A} = 1000 mg et d_{B} = 10 mg ou 5 mg
- quand B = chlorhydrate de pioglitazone :
   les doses quotidiennes requises pour A et B,
   respectivement d_{A} et d_{B} sont telles que :
   250 mg≤ d_{A} ≤ 2000 mg
   10 mg ≤ d_{B} ≤ 45 mg
   par exemple
   d_{A} = 1000 mg et d_{B} = 30 mg ou 15 mg
- quand B = maléate de rosiglitazone
   les doses quotidiennes requises pour A et B,
   respectivement d_{A} et d_{B} sont telles que :
   250 mg≤ d_{A} ≤ 2000 mg
   1 mg ≤ d_{B} ≤ 20 mg
   par exemple
   d_{A} = 1000 mg et d_{B} = 8 mg ou 4 mg
- quand B = nateglinide
   les doses quotidiennes requises pour A et B,
   respectivement d_{A} et d_{B} sont telles que :
   250 mg ≤ d_{A} ≤ 2000 mg
   100 mg ≤ d_{B} ≤ 500 mg
   par exemple
   d_{A} = 1000 mg et d_{B} = 360 mg ou 180 mg
- quand B = glipizide
   les doses quotidiennes requises pour A et B,
   respectivement d_{A} et de sont telles que :
   250 mg ≤ d_{A} ≤ 2000 mg
   2,5 mg ≤ d_{B} ≤ 40 mg
   par exemple
   d_{A} = 1000 mg et d_{B} = 15 mg ou 5 mg
- quand B = glimepiride
   les doses quotidiennes requises pour A et B,
   respectivement d_{A} et d_{B} sont telles que :
   250 mg ≤ d_{A} ≤ 2000 mg
   1 mg ≤ d_{B} ≤ 8 mg
   par exemple
   d_{A} = 1000 mg et d_{B} = 8 mg ou 4 mg

Selon encore un autre de ses objets, la présente invention concerne une méthode de traitement des diabètes de type II, dans laquelle on a recours à la forme pharmaceutique orale telle que définie ci-dessus (polythérapie, de préférence bithérapie :metformine + principe actif B).

Les exemples qui suivent permettront de mieux comprendre l'invention et d'appréhender tous ses avantages et toutes ses variantes de réalisation.

### EXEMPLES

### DESCRIPTION DES FIGURES

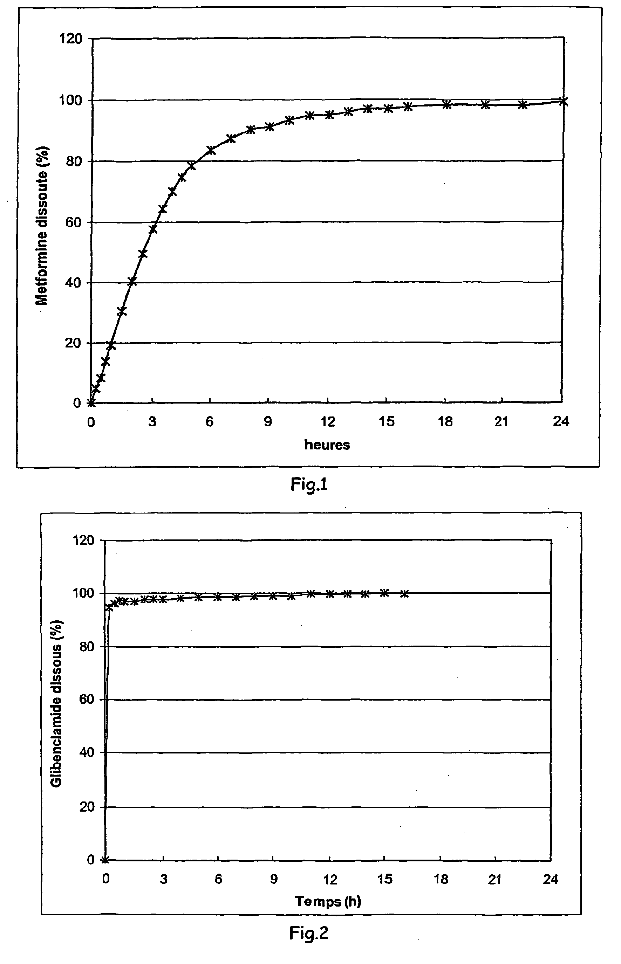

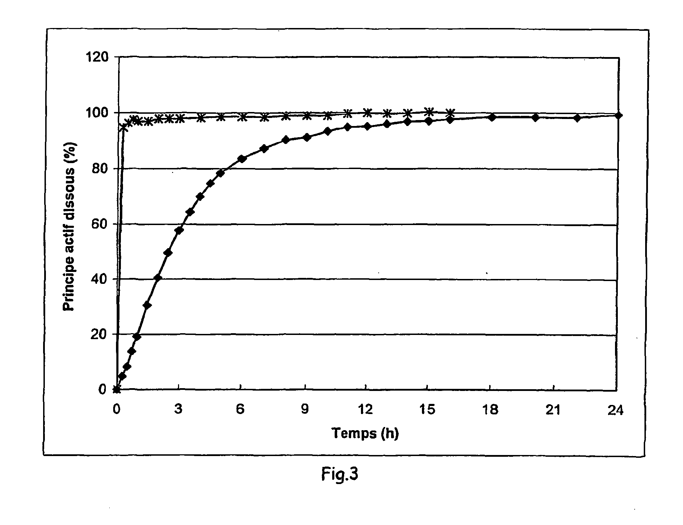

► La figure 1 représente le profil de dissolution in vitro des microcapsules de metformine selon l'exemple 1, en % de metformine dissoute en fonction du temps en heures.
► La figure 2 représente le profil de dissolution in vitro des microparticules de glibenclamide préparé selon l'exemple 2, en % de glibenclamide dissous en fonction du temps en heures.
► La figure 3 représente le profil de dissolution in vitro de chacun des deux actifs (microcapsules de metformine: -◆--◆-) / (microparticules de glibenclamide : -*--*- ), contenus dans les gélules préparées dans l'exemple 3, en % de principes actifs dissous en fonction du temps en heures.

### EXEMPLE 1 : Préparation des microcupsules de metformine

260 g d'éthylcellulose, 28 g de polyvirtylpyrrolidone, 28 g d'huile de ricin et 35 g de stéarate de magnésium sont dissous ou dispersés dans un mélange constitué de 2424 g d'acétone et 1616 g d'isopropanol. La suspension est pulvérisée sur 1000 g de cristaux de metformine/HCl, de diamètre moyen compris entre 200 et 500 µm dans un Spray coater Glatt GPC63. Les conditions de pelliculage sont : température produit : 38-42°C, vitesse de pulvérisation : 40 g/min, pression d'atomisation : 3 bars.
Les microcapsules obtenues ont été testées dans un dissolutest de type II conforme à la Pharmacopée dans un milieu tampon KH₂PO₄ / NaOH à pH 6,8, maintenu à 37°C et agité à 100 tours/min.
Le profil de dissolution obtenu est le suivent :

**TABLEAU 1**

| Temps (heure) | metformine dissoute (%) |
|---|---|
| 2 | 41 |
| 4 | 70 |
| 8 | 90 |
| 12 | 96 |
| 16 | 98 |
| 20 | 99 |

Le profil de dissolution du produit préparé dans cet exemple est représenté figure 1 annexée.

### EXEMPLE 2 : Préparation des microporticules de glibereclamide (principe actif B) à libération immédiate

312 g de polyéthylène glycol 4000, 78 g de polyvinylpyrrolidone et 43 g de glibenclamide micronisé sont dissous ou dispersés dans 2450 g d'eau. La suspension est pulvérisée sur 1300 g de microsphères de cellulose de diamètre moyen compris entre 200 et 500 µm dans un Spray coater Glatt GPCG3. Les conditions de pelliculage sont : température produit : 38-42°C, vitesse de pulvérisation : 16 g/min, pression d'atomisation : 5 bars.
Les microparticules obtenues ont été testées dans un dissolutest de type II conforme à la Pharmacopée dans un milieu tampon KH₂PO₄ / NaOH à pH 6,8, maintenu à 37°C et agité à 100 tours/min.
Le profil de dissolution obtenu est le suivent :

**TABLEAU 2**

| Temps (heure) | Glibenclamide dissous (%) |
|---|---|
| 0,25 | 95 |
| 0,5 | 96 |
| 1 | 97 |
| 2 | 98 |
| 5 | 99 |
| 12 | 100 |

Le profil de dissolution du produit préparé dans cet exemple est représenté figure 2 annexée. Ce profil est caractéristique d'une cinétique de libération immédiate.

### EXEMPLE 3 : Préparation de la forme finale gélule

Les microcapsules de metformine selon l'exemple 1 et les microparticules de glibenclamide selon l'exemple 2 sont mélangées dans le rapport 11,26 pour 1. Le mélange est mis en gélules de telle sorte que chaque gélule contient 675,5 mg de microcapsules selon l'exemple 1 et 60 mg de microparticules selon l'exemple 2, ce qui représente respectivement 500 mg de metformine, HCl et 1,5 mg de glibenclamide.
Les gélules obtenues ont été testées dans un dissolutest de type II conforme à la Pharmacopée dans un milieu tampon KH₂PO₄ / NaOH à pH 6,8, maintenu à 37°C et agité à 100 tours/min.
Le profil de dissolution obtenu pour chacun des deux actifs est conforme à celui obtenu à partir des microcapsules à base de metformine et des microparticules à base de glibenclamide, prises séparément:

**TABLEAU 3**

| Temps (heure) | Glibenclamide dissous (%) | Metformine dissoute (%) |
|---|---|---|
| 2 | 98 | 40 |
| 4 | 99 | 71 |
| 8 | 100 | 92 |
| 12 | 100 | 97 |
| 16 | 99 | 100 |
| 20 | 100 | 101 |

Le profil de dissolution pour chacun des deux actifs contenus dans les gélules préparées dans cet exemple est représenté figure 3 annexée.

### EXEMPLE 4

### 4.1

On réalise une étude pharmacocinétique comparant une forme galénique constituée par 1000 mg de metformine multimicroencapsulée selon l'exemple 1 et deux comprimés à 500 mg de metformine à libération, immédiate, commercialisés sous la marque déposée GLUCOPHAGE® (BMS/LIPHA).
Les microcapsules de metformine de l'exemple 1 et les comprimés de GLUCOPHAGE® sont administrés le soir au repas, chez 12 volontaires sains.
Des échantillons de sang sont prélevés à 0, 0,5, 1, 2, 3, 4, 5, 6, 8, 10, 12, 16, 20, 24, 36 heures après administration, pour analyse de la concentration plasmatique en metformine.
Les principaux paramètres pharmacocinétiques obtenus sont reportés ci-dessous.

**TABLEAU 4**

| | Cmax (ng/ml) | Tmax (h) | AUC₀₋ₙ (ng.ml⁻¹.h) |
|---|---|---|---|
| microcapsules metformine exemple 1 1000 mg | 996 | 7,33 | 9488 |
| GLUCOPHAGE® 500 mg x 2 | 1229 | 3,75 | 10087 |

► Tmax = temps correspondant au maximum de concentration plasmatique (Cmax) en metformine.
► AUCₒ₋ₙ = aire sous le profil de concentration plasmatique entre les instants t=O et 36 heures (biodisponibilité).

Il ressort de cette étude :
o que les microcapsules de metformine de l'exemple 1 sont
une forme pharmaceutique, dont le régime
d'administration est d'une prise par jour;
o et que le GLUCOPHAGE® 500 mg est une forme pharmaceutique, dont le régime d'administration est deux
prises par jour.

En outre, l'étude ci-dessus montre, comme cela est confirmé par l'ouvrage "Physicians" Desk Reference" - 55th edition 2001 - Ed Medical Economics Company - pages 831 to 835, que le GLUCOPHAGE® est une forme comprimé administrable deux fois par jour.

### 4.2 : Glibenclamide

L'ouvrage de référence "Physicians Desk Reference" - 54th edition 2000 - Ed Medical Economics Company - , enseigne à la page 2457 / Fig. a), que la forme galénique de comprimés à libération immédiate de glibenclamide micronisé, dosés à 3 mg et commercialisés sous la marque déposée GLYNASE PRESTAB®, est caractérisée par un régime d'administration d'une prise par jour.

### 4.3 : Gélules metformine/glibenclamide de l'exemple 3

On retrouve sur la figure 3, le même profil de libération in vitro pour la metformine que dans l'exemple 1 (Fig.1) correspondant aux seules microcapsules de metformine .
Les microcapsules de metformine de l'exemple 1 sont une forme pharmaceutique, dont le régime d'administration est d'une prise par jour (couverture thérapeutique sur 24 h.
Par conséquent, dans les gélules metformine/glibenclamide de l'exemple 3, les microcapsules de metformine assurent une couverture thérapeutique sur 24 h.

On retrouve sur la figure 3, le même profil de libération in vitro pour le glibenclamide, que dans l'exemple 2 (Fig.2) correspondant aux seules microparticules de glibenclamide.

Les gélules de l'exemple 3 présentent donc les profils in vitro adéquats pour une forme à libération prolongée, assurant une couverture thérapeutique sur 24 h, pour la metformine et le glibenclamide.

## Revendications

1. Forme pharmaceutique orale associant :
➢ un principe actif A constitué par un biguanide, à savoir la metformine,
➢ et au moins un autre principe actif B différent de A et choisi parmi les antidiabétiques, de préférence les antihyperglycémiants, dont le régime d'administration est d'une ou plusieurs prises par jour,
**caractérisée**
**❖ en ce qu'**elle comporte :
• une pluralité de capsules constituées chacune par un coeur à base de metformine et par une pellicule d'enrobage appliquée sur le coeur et permettant la libération prolongée in vivo de la metformine ;
• et éventuellement dans le cas où le régime d'administration du principe actif B est égal à plusieurs prises par jour, une pluralité de capsules constituées chacune par un coeur à base de principe actif B et par une pellicule d'enrobage appliquée sur le coeur et permettant la libération prolongée in vivo du principe actif B ;
❖ et en ce que les capsules à base de A et les éventuelles capsules à base de B, sont conçues de telle sorte que le régime d'administration de la forme galénique considérée soit d'une prise par jour :
**caractérisée en ce que** les capsules ont une granulométrie comprise entre 50 et 1000 µm, de préférence entre 100 et 750 µm, et, plus préférentiellement encore, entre 200 et 500 µm ;
et **caractérisée en ce que** la composition de la pellicule d'enrobage des capsules est la suivante :
1) - au moins un polymère filmogène (P1) insoluble dans les liquides du tractus, présent à raison de 50 à 90, de préférence 50 à 80 % en poids sur sec par rapport à la masse totale de la composition d'enrobage et constitué par au moins un dérivé non hydrosoluble de la cellulose, à savoir l'éthylcellulose et/ou l'acétate de cellulose.
2) - au moins un polymère azoté (P2) présent à raison de 2 à 25, de préférence 5 à 15 % en poids sur sec par rapport à la masse totale de la composition d'enrobage et constitué par au moins un polyacrylamide et/ou un poly-N-vinylamide et/ou un poly-N-vinyl-lactame, à savoir le polyacrylamide et/ou la polyvinylpyrrolidone ;
3) - au moins un plastifiant présent à raison de 2 à 20, de préférence de 4 à 15 % en poids sur sec par rapport à la masse totale de la composition d'enrobage et constitué par au moins l'un des composés suivants : les esters du glycérol, les phtalates, les citrates, les sébaçates, les esters de l'alcool cétylique; l'huile de ricin ;
4) - et éventuellement au moins un agent tensioactif et/ou lubrifiant, présent à raison de 2 à 20, de préférence de 4 à 15 % en poids sur sec par rapport à la masse totale de la composition d'enrobage et choisi parmi les tensioactifs anioniques, à savoir les sels alcalins ou alcalinoterreux des acides gras, l'acide stéarique et/ou oléique étant préférés, et/ou parmi les tensioactifs non ioniques, à savoir les esters de sorbitan polyoxyéthylénés et/ou les dérivés de l' huile de ricin polyoxyéthylénés, et/ou parmi les agents lubrifiants comme les stéarates de calcium, de magnésium, d'aluminium ou de zinc, ou comme le stéarylfumarate de sodium et/ou le béhénate de glycérol; ledit agent pouvant comprendre un seul ou un mélange des susdits produits.

2. Forme pharmaceutique orale selon la revendication 1, **caractérisée en ce que** l'enrobage des capsules à base de A et des éventuelles capsules à base de B, a la composition suivante :
-1- Ethylcellulose
-2- Polyvinylpyrrolidone
-3- Huile de ricin
-4- Stéarate de magnésium.

3. Forme pharmaceutique orale selon l'une quelconque des revendications 1 à 2, **caractérisée en ce que** le (ou les) principe(s) actif(s) B est (sont) choisi(s) dans le groupe de familles d'agents antihyperglycémiants comprenant :
• LES SULFONYLUREES ;
le glibenclamide, le nateglinide, le glimepiride, le glipizide, le gliclazide,
le tolbutamide, le tolazamide, la gliquidone et le chlorpropamide étant
plus spécifiquement retenus ;
• LES THIAZOLIDINEDIONES :
le maléate de rosiglitazone, la troglitazone, le zorglitazone, l'englitazone, la dorglitazone, le produit MITSUBISHI MCC-555, le produit GLAXO-WELCOME GL-262570 et le chlorhydrate de pioglitazone étant plus spécifiquement retenus :
• LES INHIBITEURS D'α-GLUCOSIDASE ;
l'acarbose ou le miglitol ;
• LES METIGLINIDES :
le repaglinide :
• LES INSULINES :
• ET LEURS ASSOCIATIONS.

4. Forme pharmaceutique orale selon l'une quelconque des revendications 1 à 3, **caractérisée en ce qu'**elle se présente sous forme d'une unité galénique de masse et de volume adaptés pour permettre à chaque administration orale journalière, l'absorption des doses respectives d_{A} et d_{B} quotidiennes requises en principes actifs A et B.

5. Forme pharmaceutique orale selon l'une quelconque des revendications 1 à 4, **caractérisée en ce qu'**elle est constituée par un comprimé, une gélule ou de la poudre.

6. Forme pharmaceutique orale selon l'une quelconque des revendications 1 à 5, **caractérisée en ce que :**
• quand B = glibenclamide :
les doses quotidiennes requises pour A et B,
respectivement d_{A} et d_{B} sont telles que :
250 mg≤ d_{A} ≤ 2000 mg
1,25 mg≤ d_{B} ≤ 20 mg
• quand B = chlorhydrate de pioglitazone :
les doses quotidiennes requises pour A et B,
respectivement d_{A} et d_{B} sont telles que :
250 mg≤ d_{A} ≤ 2000 mg
10 mg≤ d_{B} ≤ 45 mg
• quand B = maléate de rosiglitazone
les doses quotidiennes requises pour A et B, respectivement d_{A} et d_{B} sont telles que :
250 mg≤ d_{A} ≤ 2000 mg
1 mg ≤ d_{B} ≤ 20 mg
• quand B = nateglinide
les doses quotidiennes requises pour A et B, respectivement d_{A} et d_{B} sont telles que :
250 mg ≤ d_{A} ≤ 2000 mg
100 mg ≤ d_{B} ≤ 500 mg
• quand B = glipizide
les doses quotidiennes requises pour A et B, respectivement d_{A} et d_{B} sont telles que :
250 mg ≤ d_{A} ≤ 2000 mg
2,5 mg ≤ d_{B} ≤ 40 mg
• quand B = glimepiride
les doses quotidiennes requises pour A et B, respectivement d_{A} et d_{B} sont telles que :
250 mg ≤ d_{A} ≤ 2000 mg
1 mg ≤ d_{B} ≤ 8 mg.

## Claims

1. Oral pharmaceutical form combining:
➢ an active ingredient A constituted by a biguanide, namely metformin,
➢ and at least one other active ingredient B which is different from A and chosen from the antidiabetics, preferably the antihyperglycemics, the dosing regimen of which is one or more doses per day,
**characterized**
**❖ in that** it comprises:
• a plurality of capsules each constituted by a core based on metformin and a coating film which is applied to the core and which allows the *in-vivo* sustained release of the metformin;
• and optionally, where the dosing regimen of the active ingredient B is equal to several does per day, a plurality of capsules each constituted by a core based on active ingredient B and a coating film which is applied to the core and which allows the *in-vivo* sustained release of the active ingredient B;
❖ and in that the capsules based on A and the optional capsules based on B are designed in such a way that the dosing regimen of the pharmaceutical form under consideration is one dose per day.
**characterized in that** the capsules have a particle size comprised between 50 and 1000 µm, preferably between 100 and 750 µm and even more preferably between 200 and 500 µm;
and **characterized in that** the coating film is as follows:
1)- at least one film-forming polymer (P1) which is insoluble in the fluids of the tract, present at a rate of 50 to 90, preferably 50 to 80 % by dry weight with respect to the total mass of the coating composition and constituted by at least one water-insoluble cellulose derivative, namely ethyl cellulose and/or cellulose acetate;
2)- at least one nitrogenous polymer (P2) present at a rate of 2 to 25, preferably 5 to 15 % by dry weight with respect to the total mass of the coating composition and constituted by at least one polyacrylamide and/or a poly-N-vinylamide and/or a poly-N-vinyl-lactam, namely polyacrylamide and/or polyvinylpyrrolidone;
3)- at least one plasticizer present at a rate of 2 to 20, preferably 4 to 15 % by dry weight with respect to the total mass of the coating composition and constituted by at least one of the following compounds: glycerol esters, phthalates, citrates, sebacates, cetylic alcohol esters, castor oil;
4)- and optionally at least one surfactant and/or lubricant, present at a rate of 2 to 20, preferably 4 to 15 % by dry weight with respect to the total mass of the coating composition and chosen from the anionic surfactants, namely the alkaline or alkaline earth salts of fatty acids, stearic and/or oleic acid being preferred, and/or from the non-ionic surfactants, namely the polyoxyethylenated sorbitan esters and/or the polyoxyethylenated castor oil derivatives, and/or from the lubricants such as calcium, magnesium, aluminium or zinc stearates, or such as sodium stearyl fumarate and/or glycerol behenate; said agent being able to comprise just one or a mixture of the above-mentioned products.

2. Oral pharmaceutical form according to claim 1, **characterized in that** the coating of the capsules based on A and the optional capsules based on B has the following composition:
-1- Ethyl cellulose
-2- Polyvinylpyrrolidone
-3- Castor oil
-4- Magnesium stearate.

3. Oral pharmaceutical form according to any one of claims 1 to 2, **characterized in that** the active ingredient(s) B is (are) chosen from the group of families of antihyperglycemics comprising:
• THE SULPHONYLUREAS:
glibenclamide, nateglinide, glimepiride, glipizide, gliclazide, tolbutamide, tolazamide, gliquidone and chlorpropamide being more specifically retained;
• THE THIAZOLIDINEDIONES:
rosiglitazone maleate, troglitazone, zorglitazone, englitazone, darglitazone, the product MITSUBISHI MCC-555, the product GLAXO-WELCOME GL-262570 and pioglitazone hydrochloride being more specifically retained;
• THE α-GLUCOSIDASE INHIBITORS:
acarbose or miglitol;
• THE METIGLINIDES:
repaglinide;
• THE INSULINS;
• AND COMBINATIONS THEREOF.

4. Oral pharmaceutical form according to any one of claims 1 to 3, **characterized in that** it is present in the form of a pharmaceutical unit with a mass and a volume that are adjusted to allow, at each daily oral administration, the absorption of the required daily doses d_{A} and d_{B} of active ingredients A and B respectively.

5. Oral pharmaceutical form according to any one of claims 1 to 4, **characterized in that** it is constituted by a tablet, a gelatine capsule or powder.

6. Oral pharmaceutical form according to any one of claims 1 to 5, **characterized in that:**
• when B = glibenclamide:
the daily doses required for A and B,
d_{A} and d_{B} respectively are such that:
250 mg ≤ d_{A} ≤ 2000 mg
1.25 mg ≤ d_{B} ≤ 20 mg
• when B = pioglitazone hydrochloride:
the daily doses required for A and B,
d_{A} and d_{B} respectively are such that:
250 mg ≤ d_{A} ≤ 2000 mg
10 mg ≤ d_{B} ≤ 45 mg
• when B = rosiglitazone maleate
the daily doses required for A and B,
d_{A} and d_{B} respectively are such that:
250 mg ≤ d_{A} ≤ 2000 mg
1 mg ≤ d_{B} ≤ 20 mg
• when B = nateglinide
the daily doses required for A and B, d_{A} and d_{B} respectively are such that:
250 mg ≤ d_{A} ≤ 2000 mg
100 mg ≤ d_{B} ≤500 mg
• when B = glipizide
the daily doses required for A and B, d_{A} and d_{B} respectively are such that:
250 mg ≤ d_{A} ≤ 2000 mg
2.5 mg ≤ d_{B} ≤ 40 mg
• when B = glimepiride
the daily doses required for A and B, d_{A} and d_{B} respectively are such that:
250 mg ≤ d_{A} ≤ 2000 mg
1 mg ≤ d_{B} ≤ 8 mg.

## Patentansprüche

1. Orale pharmazeutische Darreichungsform, in der folgendes gemeinsam vorliegt:
➢ ein Wirkprinzip A, das aus einem Biguanid, nämlich Metformin, gebildet wird,
➢ und mindestens ein weiteres Wirkprinzip B, das sich von A unterscheidet und das aus der Gruppe der Antidiabetika, vorzugsweise der Antihyperglykämika, deren Verabreichungsschema eine ein- oder mehrmalige Einnahme pro Tag ist, ausgewählt ist,
**dadurch gekennzeichnet,**
❖ **daß** sie folgendes umfaßt:
■ eine Mehrzahl von Kapseln, die jeweils aus einem Kern auf Basis von Metformin und einem auf dem Kern aufgebrachten Hüllfilm gebildet werden und die die protrahierte Freigabe von Metformin in vivo gestatten;
■ sowie gegebenenfalls, wenn das Verabreichungsschema des Wirkprinzips B mehrere Einnahmen pro Tag ist, eine Mehrzahl von Kapseln, die jeweils aus einem Kern auf Basis von Wirkprinzip B und einem auf den Kern aufgebrachten Hüllfilm der die protrahierte Freigabe von Wirkprinzip B in vivo gestattet, gebildet werden;
❖ und daß die Kapseln auf Basis von A und die gegebenenfalls vorliegenden Kapseln auf Basis von B dergestalt sind, daß das Verabreichungsschema der jeweiligen galenischen Form eine Einnahme pro Tag beträgt;
**dadurch gekennzeichnet, daß** die Kapseln eine Granulometrie zwischen 50 und 1000 µm, vorzugsweise zwischen 100 und 750 µm, noch stärker bevorzugt zwischen 200 und 500 µm aufweisen;
sowie **dadurch gekennzeichnet, daß** die Zusammensetzung des Hüllfilms der Kapseln folgendermaßen lautet:
1)- mindestens ein in Verdauungssäften unlösliches filmbildendes Polymer (P1), das in einer Menge von 50 bis 90, vorzugsweise 50 bis 80 Gew.-%, in der Trockenmasse in Bezug auf die Gesamtmasse der Hüllzusammensetzung vorliegt und das aus mindestens einem nichtwasserlöslichen Cellulosederivat, nämlich Ethylcellulose und/oder Celluloseacetat gebildet wird,
2)- mindestens ein stickstoffhaltiges Polymer (P2), das in einer Menge von 2 bis 25, vorzugsweise 5 bis 15 Gew.-%, in der Trockenmasse in Bezug auf die Gesamtmasse der Hüllzusammensetzung vorliegt und das aus mindestens einem Polyacrylamid und/oder einem Poly-N-vinylamid und/oder einem Poly-N-vinyllactam, nämlich Polyacrylamid und/oder Polyvinylpyrrolidon, gebildet wird;
3)- mindestens ein Weichmacher, der in einer Menge von 2 bis 20, vorzugsweise 4 bis 15 Gew.-%, in der Trockenmasse in Bezug auf die Gesamtmasse der Hüllzusammensetzung vorliegt und aus mindestens einer der folgenden Verbindungen gebildet wird: Glycerolester, Phthalate, Citrate, Sebacate, Cetylalkoholester, Rizinusöl;
4)- sowie gegebenenfalls mindestens ein Tensid und/oder Gleitmittel, das in einer Menge von 2 bis 20, vorzugsweise 4 bis 15 Gew.-%, in der Trockenmasse in Bezug auf die Gesamtmasse der Hüllzusammensetzung vorliegt und das aus der Reihe der anionischen Tenside, nämlich der Alkali- oder Erdalkalisalze der Fettsäuren, vorzugsweise Stearinsäure und/oder Ölsäure und/oder aus der Reihe der nichtionischen Tenside, nämlich der polyoxyethylierten Sorbitanester und/oder der polyoxyethylierten Rizinusölderivate, und/oder aus der Reihe der Gleitmittel wie Kalziumstearat, Magnesiumstearat, Aluminiumstearat oder Zinkstearat, oder wie Natriumstearylfumarat und/oder Glycerolbehenat ausgewählt ist, wobei dieses Mittel eines oder eine Mischung der obengenannten Produkte umfassen kann,

2. Orale pharmazeutische Darreichungsform nach Anspruch 1, **dadurch gekennzeichnet, daß** die Umhüllung der Kapseln auf Basis von A und der gegebenenfalls vorhandenen Kapseln auf Basis von B die folgende Zusammensetzung aufweist:
-1- Ethylcellulose
-2- Polyvinylpyrrolidon
-3- Rizinusöl
-4- Magnesiumstearat.

3. Orale pharmazeutische Darreichungsform nach einem der Ansprüche 1 bis 2, **dadurch gekennzeichnet, daß** das Wirkprinzip B bzw. die Wirkprinzipien B aus der Gruppe der Familie der Antihyperglykämika, umfassend:
• SULFONYLHARNSTOFFE, unter besonderer Beachtung von Glibenclamid, Nateglinid, Glimepirid, Glipizid, Gliclazid, Tolbutamid, Tolazamid, Gliquidon und Chlorpropamid,
• THIAZOLIDINDIONE, unter besonderer Beachtung von Rosiglitazonmaleat, Troglitazon, Zorglitazon, Englitazon, Darglitazon, des Produkts MITSUBISHI MCC-555, des Produkts GLAXO-WELCOME GL-262570 und Pioglitazon-hydrochlorid;
• α-GLUCOSIDASEHEMMER; Acarbose oder Miglitol;
• METIGLINIDE; Repaglinid;
• INSULINE;
• UND KOMBINATIONEN DAVON
ausgewählt ist/sind.

4. Orale pharmazeutische Darreichungsform nach einem der Ansprüche 1 bis 3, **dadurch gekennzeichnet, daß** sie in Form einer galenischen Einheit vorliegen, deren Masse und Volumen dergestalt sind, daß sie bei jeder oralen Verabreichung pro Tag die Absorption der jeweils erforderlichen Tagesdosen d_{A} bzw. d_{B} am Wirkprinzip A und B gestatten.

5. Orale pharmazeutische Darreichungsform nach einem der Ansprüche 1 bis 4, **dadurch gekennzeichnet, daß** sie aus einer Tablette, einer Gelatinekapsel oder Pulver besteht.

6. Orale pharmazeutische Darreichungsform nach einem der Ansprüche 1 bis 5, **dadurch gekennzeichnet, daß:**
• wenn B = Glibenclamid,
die für A und B jeweils erforderlichen Tagesdosen d_{A} und d_{B} folgendermaßen lauten:
250 mg ≤ d_{A} ≤ 2000 mg
1,25 mg ≤ d_{B} ≤ 20 mg
• wenn B = Pioglitazon-hydrochlorid,
die für A und B jeweils erforderlichen
Tagesdosen d_{A} und d_{B} folgendermaßen lauten:
250 mg ≤ d_{A} ≤ 2000 mg
10 mg ≤ d_{B} ≤ 45 mg
• wenn B = Rosiglitazonmaleat,
die für A und B jeweils erforderlichen
Tagesdosen d_{A} und d_{B} folgendermaßen lauten:
250 mg ≤ d_{A} ≤ 2000 mg
1 mg ≤ d_{B} ≤ 20 mg
• wenn B = Nateglinid,
die für A und B jeweils erforderlichen
Tagesdosen d_{A} und d_{B} folgendermaßen lauten:
250 mg ≤ d_{A} ≤ 2000 mg
100 mg ≤ d_{B} ≤ 500 mg
• wenn B = Glipizid,
die für A und B jeweils erforderlichen
Tagesdosen d_{A} und d_{B} folgendermaßen lauten:
250 mg ≤ d_{A} ≤ 2000 mg
2, 5 mg ≤ d_{B} ≤ 40 mg
• wenn B = Glimepirid,
die für A und B jeweils erforderlichen
Tagesdosen d_{A} und d_{B} folgendermaßen lauten:
250 mg ≤ d_{A} ≤ 2000 mg
1 mg ≤ d_{B} ≤ 8 mg.
